(19) Europäisches Patentamt

European Patent Office

Office européen des brevets

(11) EP 1 366 782 A1

(12) **EUROPEAN PATENT APPLICATION**

(43) Date of publication:
**03.12.2003 Bulletin 2003/49**

(51) Int Cl.⁷: $A61N\ 2/02$, $A61N\ 2/00$

(21) Application number: **03396048.5**

(22) Date of filing: **26.05.2003**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **31.05.2002 FI 20021050**

(71) Applicant: **Nexstim Oy**
**FIN-00510 Helsinki (FI)**

(72) Inventor: **Ruohonen, Jarmo**
**01650 Vantaa (FI)**

(74) Representative: **Lipsanen, Jari Seppo Einari et al**
**Seppo Laine Oy,**
**Itämerenkatu 3 B**
**00180 Helsinki (FI)**

(54) **Targeting method and apparatus for the magnetic stimulation of the brain**

(57) The invention relates to a method and apparatus for targeting electromagnetic stimulation on the human brain. According to the method, pulse-like electromagnetic fields are formed, which have a strong local electromagnetic maximum, the electromagnetic maximum of the field is targeted on the brain (2), the distribution, direction, and strength of the electromagnetic field arising in the brain are defined, and some physiological or biological response, such as, for example, a muscular response measured using electromyography, is measured. According to the invention, the position of the brain is defined in three-dimensional space and the position data is received in electrical form in a data-processing apparatus (8), the position data of the electromagnetic fields are received in the data-processing apparatus (8) in the same three-dimensional set of coordinates as the brain, the position data of the electromagnetic fields are combined with the position data of the brain (2), and a graphical presentation (9) of the position data of the electromagnetic fields and of the position data of the brain is formed with the aid of the data-processing apparatus (8).

EP 1 366 782 A1

## Description

The present invention relates to a method according to the preamble of Claim 1 and an apparatus according to the preamble of Claim 12.

Methods and apparatuses of this kind are used for the measurement of and research into biological tissue, and for therapy by stimulating the tissue electromagnetically.

A suitable electric field can stimulate the human brain and the peripheral nerves or muscles. In magnetic stimulation, an electric field is induced painlessly and safely by means of a variable magnetic field. In magnetic stimulation, a coil made of electrically conductive material is placed above the target tissue. A powerful pulse of electrical current, the duration of which is typically 100 - 1000 µs, is led through the coil. A varying magnetic field arises around the coil, with a magnitude of up to 3 Teslas, and which in accordance with Faraday's Law induces an electric field in the electrically conductive tissue in the vicinity of the coil. The magnetic and electric fields weaken rapidly as the distance from the coil increases. In stimulation, the coil is generally placed as close to the target as possible.

The coil used in magnetic stimulation typically has a diameter of 50 - 100 mm and consists of 10 - 30 concentric turns of copper wire. The coil can have, for example, a round or figure-of-eight shape. The coil may also have a ferromagnetic core. The shape and force of the stimulating electric field depend on, among other factors, the shape of the coil and its position relative to the target. The shape and electrical conductivity geometry of the target tissue also strongly affect the characteristics of the stimulating field. The direction of the electric field in the tissue being irritated also affects which parts of the tissue are most strongly irritated.

Magnetic stimulation is used for several different purposes. If the electric field hits the 1st somatosensoric area of the brain cortex controlling the movements of the hand, for example, contraction of the muscles of the hand can be detected after a short delay. This can be exploited when determining the functionality of the nerves between the brain and the muscles. By stimulating the brain, it is also possible to disturb its normal operation, for instance, during some task, so that it is possible to determine which parts of the brain are important for the carrying out the task in question. It has also been reported that magnetic stimulation has therapeutic effects on patients suffering from depression.

In several applications of magnetic stimulation, the objective is to stimulate a predefined part of the brain cortex. A suitable place is found by placing the coil manually in different locations and attitudes, until the desired effect of the magnetic stimulation is detected. After this, the coil is kept in the same place for the entire investigation. It is also often necessary to be able to know, after the stimulation, what location in the brain was stimulated by the coil placed against the head. Similarly, it is necessary to define the relation to the stimulated location of the magnitude of some measured biological or physiological response. This necessary in, for example, brain research, in which the relationships between stimulation and its effects are studied, in order to determine the functioning of the brain.

According to the prior art, the magnetic stimulation is targeted by placing the coil on a suitable location on the head. The location of the coil is defined by measuring the distance of its centrepoint from a selected fixed point on the head, such as the auditory canal. The location can also be defined in relation to the location of the coil that causes a detectable contraction in, for instance, the muscles of the thumb of the right hand. According to the prior art, it is also possible to attach positioning sensors to the coil and the head and to position the location of the coil relative to the head. According to the prior art, magnetic images taken of the head of an experimental subject or patient can also be used to show the location of the coil relative to the anatomical structures located in the magnetic image. Such an application is referred to in, *inter alia,* the publication Ruohonen & Ilmoniemi, *Medical & Biological Engineering & Computing,* 36:297 - 301, 1996.

A drawback in the prior art is that different kinds of coils induce different kinds of electromagnetic fields in the head. The location of the maximum effect of the field may vary by up to 5 cm, depending on the shape of the coil. In methods according to the prior art, no allowance is made for the fact that the electrical field induced in the brain is generally not beneath the centre point of the coil. Methods according to the prior art also do not allow for the fact that the electromagnetic field inside the head changes significantly, if the coil is tilted relative to the head, even if the location of the coil remains the same.

A second drawback is that fact that, in methods according to the prior art, the size of the stimulating field striking the various parts of the brain is not known. The stimulation of several parts of the brain cortex does not give rise to easily detectable physiological effects. It is thus a problem to create stimulation of a desired magnitude in these areas of the brain cortex. The strength of the stimulation depends greatly on the distance of the coil from the brain cortex and a difference of even one millimetre in the distance can significantly change the stimulating power of the field on the brain cortex. There is also the problem that the distance of the cortex from the surface of the scalp varies in different people.

A third problem is the fact that, when seeking for the location and attitude in which to place the coil, so that it will create, for example, the greatest muscular response in the muscles of one thumb, it is difficult, or nearly impossible for the operator to set the coil in precisely the same place on several different occasions. When it is wished to stimulate precisely the same point for a period of several minutes or even tens of minutes, the location or attitude of the coil in relation to the head can easily change, as the coil cannot be firmly attached to the head or the scalp. There is then no way for the operator to know if the stimulating field is the same and is in the same place for the entire duration of the experiment.

The invention is based on the position data of both the coil and the head being transferred to a data system, with the aid of which a visual model is created for controlling the operation of the apparatus in three-dimensional space. The operation preferably takes place in at least more or less real time.

More specifically, the method according to the invention is characterized by what is stated in the characterizing portion of Claim 1.

The apparatus according to the invention is, in turn, characterized by what is stated in the characterizing portion of Claim 12.

Considerable advantages are gained with the aid of the invention.

One advantage is that the operator can plan the operation precisely, so that the electrical field induced by the magnetic stimulation is strongest at exactly the point in the brain cortex that they desire and that the direction of the electrical field is that desired.

A second advantage is that, in the method, it is possible to define the dependence relationship between the stimulation and the measured or the detected response. For example, it is possible to draw a chart, showing the magnitude of the measured response at that point in the brain cortex, at which the most powerful electrical field is targeted. The magnitude of the measured response can also be shown corrected by a coefficient depicting the power of the electrical field. The response can also be used as feedback when defining the power or hit-area of the stimulation.

A third advantage is that the same area of the brain cortex can be stimulated, easily and reliably, in the same person, using a precisely similar electrical field, on different days, or even in different years.

The invention is next examined with the aid of examples of embodiments according to the accompanying drawings.

Figure 1 shows a head image according to the invention.

Figure 2 shows a brain image according to the invention.

Figure 3 shows a top view of the location of the coil in the method according to the invention.

Figure 4 shows a schematic side view of the system according to the invention.

The invention thus refers to a computer-controlled method, in which the operator is guided interactively to position the coil at precisely the location and the angle of tilt, at which the coil will stimulate most strongly the target point in the brain selected by the operator. Generally, the target point can also be selected from magnetic-resonance images, which can be browsed on the display of the computer. The operator can also select the direction from which they wish the electrical field to strike the target.

The target point can also be selected on the basis of some operational data, by entering the co-ordinates of the point in a computer program. The target point can also be, for instance, a function previously positioned by magnetic stimulation. In the method according to the invention, the position of the head relative to the coil is measured several times a second and the position of the coil is displayed numerically or graphically to the operator. The computer simultaneously calculates an estimate of the electrical field created in the brain by the coil, if a stimulation pulse were to be released from the position of the coil at that moment. The electrical field is displayed to the operator numerically and graphically. If the coil is moved on top of the head, the display thus shows a real-time estimate of the characteristics in the brain of the stimulating field created by the coil. On the basis of the data given to the operator in real time, they can set the coil in a location and attitude that they have predefined.

The method to which the invention refers can also include the possibility to record, after every stimulation pulse, the position of the coil and its attitude relative to the tissue (such as the head) being stimulated. As the experiment proceeds, or after it the position of the coil and the electrical field created by the stimulation pulse are shown to the operator on the display of the computer. On the basis of the data provided, the operator can ascertain whether each stimulation given during the experiment hit the desired target, with the desired strength and in the desired direction.

In the method according to the invention, some part of the brain is stimulated by magnetic stimulation while the position of the stimulation coil and some physiological response are simultaneously measured. When the operator gives a stimulation pulse, the position of the coil is recorded and an estimate, including the distribution, strength, and direction, of the electrical field created in the brain, is calculated. An illustrative chart or other graphical presentation is drawn from the results, and shows the distribution and strength of the electrical field, as well as the relationship with a physiological response.

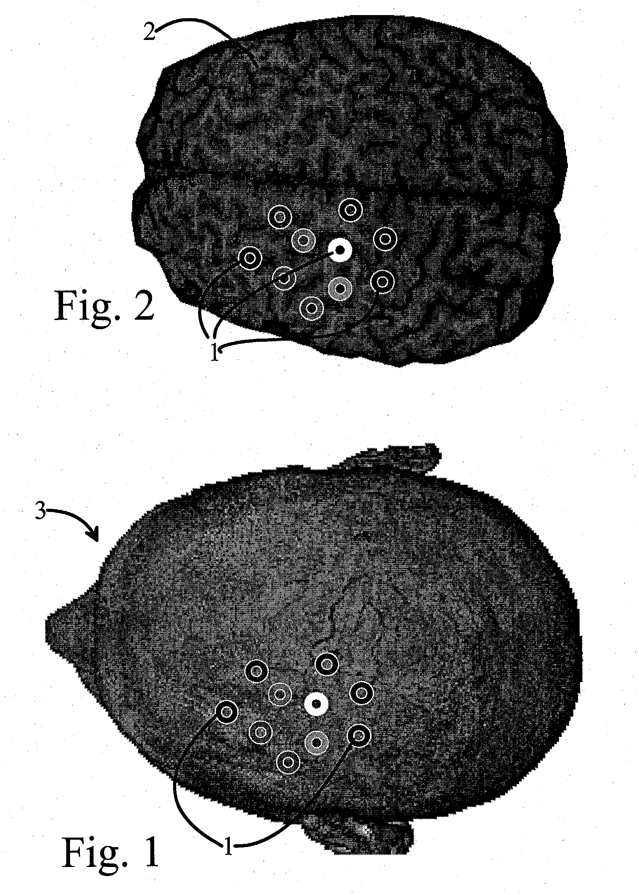

Figure 1 shows one illustrative manner of visualization. In the figure, the positioning points of the coil on the scalp and the points of maximum effect created by the coil in the brain cortex are drawn as circles 1. The grey tones of the rings depict the magnitude of the measured physiological response using the relevant placing of the coil. The manner of illustration may also be drawing a set of level-curves, or the numerical presentation of numerical values. In Figure 2, the same points are shown on the surface of the brain 2.

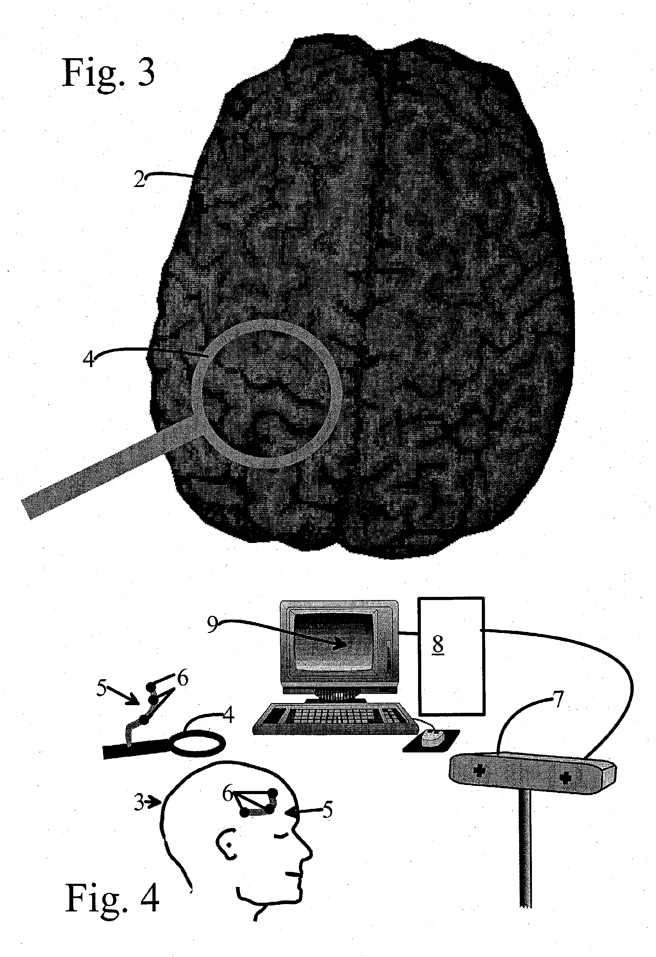

With reference to Figures 3 and 4, the invention is based on the location and direction of the coil 4 relative to the head 3 being measured precisely using a positioning device 7. More specifically, a position sensor 5, which measures the attitude and location of the head, is attached to the head of the test subject. The location of the sensor 5 relative to fixed points (at least 3 of them) on the head 3 is determined by measuring their location with the positioning device 7. Corresponding fixed points are sought from the magnetic image of the head, so that a co-ordinate conversion between the position sensor and the magnetic image can be created. When the coil 4, to which a second position sensor 5 is attached, is now placed on top of the head 3, the position and attitude of the coil 4 relative to the position sensor 5 can be precisely defined. Using the aforesaid coordination conversion, it is then possible to show the location and attitude of the coil 4 relative to any chosen point in the magnetic image. A group of balls 6 reflecting infrared light, for example, can act as the position sensor. When a pulse of infrared light is sent towards the balls 6, they reflect it. By using a suitably precise measuring device 7, it is possible to detect the reflected light and position the balls. If there are at least three balls 6 in the group, and their position relative to each other is known, the precise location and attitude of the group of balls can be determined. Measuring devices of this type are available from at least the Canadian company Northern Digital Inc. The location and attitude data of the sensors 5 is transferred from the positioning device to the computer 8 and presented in a suitable format on, for example, the display 9. The display 9 can naturally be any kind of display, such as a conventional cathode-ray tube, a liquid-crystal display, a plasma display, etc. The use of colour in the display will improve the illustrative quality of the image shown.

The position sensor 5 of the coil 4 should be attached firmly to the coil 4 and its exact location and attitude relative to the structure of the coil should be known. The location and attitude of the sensor 5 can be defined using the same positioning device 7, by bringing the coil to some known attitude and location. The positioning device 7 can then be used to determine the position of the sensor 5 attached to the coil and its attitude relative to the positioning device, and the location of the sensor 5 relative to the coil 4 can be calculated.

The electrical field induced in the brain can be calculated precisely, if the characteristics of the stimulation pulse and the location and attitude of the coil 4 relative to the head 3 are known. The electrical field **E** is a vector, which can be calculated in a known manner for a moment t in time, from the formula:

$$E(X,Y,Z,t) = -\partial A(X,Y,Z,t)/ \partial t - \nabla V(X,Y,Z,t).$$

The above vector potential A of the coil is calculated according to the literature dealing with electromagnetic fields. The calculation requires data on the coil's geometry, attitude, and location relative to the point X,Y,Z and on the characteristics of the current pulse sent through the coil. The electrical potential V is also calculated using methods known from the literature, as a solution of the Laplace equation $\Delta^2V = 0$. The solution requires knowledge of the conductivity geometry of the head. The most important calculation results are obtained by using the element method in the calculation and the electrical conductivity at different points in the head derived from the magnetic image.

The invention is based on the fact that, by combining the calculation of the electrical field and the positioning of the coil, the coil can be easily and precisely placed on a point on top of the head, at which the maximum magnetic stimulation will be targeted at the desired target point. The invention is essentially associated with a computer program, with the aid of which the operator of the device can set the target point interactively in the magnetic image. At the same time, the computer program is interactive and visualizes graphically or numerically the location and strength of the maximum point of the coil and the electrical field that it creates.

In stereotactic targeting, the computer guides the operator to bring the coil to the location and attitude above the head, at which the electrical field that it creates in the brain is strongest at the target point preselected by the operator. If the coil is positioned in real time using a positioning system and the electrical field it creates is also calculated in real time, the operator will be able to visualize illustratively and interactively, the direction in which they should move or tilt the coil, in order to obtain maximum stimulation at the target point.

Correspondingly, precise charts can be formed of the point at which stimulation causes a specific detectable or measurable biological or physiological response. Such a response can be, for example, an electrical muscular response caused by stimulation of the motoric brain cortex. If magnetic stimulation is used to disturb brain activity during a specific task, the response can be visualized as being how strongly the stimulation disturbs the relevant task.

An alternative embodiment is that, according to the invention, the operator searches for the target point from the magnetic image, with the aid of a computer program. After that, the computer automatically seeks for the co-ordinates of the surface of the scalp from the magnetic image and for the point on the scalp from which there is the shortest distance to the target point. The coil should then be set in such a way that its focal point corresponds to the relevant point on the scalp. The focal point is the point on the coil, which, when placed on top of the head, creates the strongest electrical current beneath it; it can be easily defined by calculation according to known methods. For example, the focal point of a figure-of-eight coil is, as is known, its centre point. The operator can also be guided to tilt the coil in such a way that the focal point moves to the desired location.

A second alternative embodiment is one in which the device includes a robot, which takes the coil automatically to the point at which, when it is set there, it causes the maximum stimulation at the target point defined by the operator.

Alternatively, instead of the operator being shown the magnitude of the electrical field induced in the brain cortex, they can be shown only the point, at which the field is maximum. In addition, the magnitude of the relevant field can also be displayed.

In the present application, the term 'at least more or less real-time operation' refers to an operating speed of the apparatus, at which the location of the coil can be easily illustrated and fine-adjusted with the aid of the display 9. In practice, delays in operation of this kind can be at most a few seconds.

**Claims**

1. A method for targeting electromagnetic stimulation on the human brain, in which method

    - pulse-like electromagnetic fields, which have a strong local electromagnetic maximum, are formed,
    - the electromagnetic maximum of the field is targeted on the brain (2),
    - the distribution, direction, and strength of the electromagnetic field arising in the brain are defined, and
    - some physiological or biological response, such as, for example, a muscular response measured using electromyography, is measured,

    **characterized in that**

    - the position of the brain is defined in three-dimensional space and the position data is received in electrical form in a data-processing apparatus (8),
    - the position data of the electromagnetic fields is received in the data-processing apparatus (8), in electrical form, in at least more or less real time,
    - the positions of the electromagnetic fields are modelled in the same three-dimensional set of co-ordinates as the position of the brain (2), with the aid of the data-processing apparatus (8),
    - the position data of the electromagnetic fields are combined with the position data of the brain (2), and
    - a graphical presentation (9) of the position data of the electromagnetic fields and of the position data of the brain is formed with the aid of the data-processing apparatus (8).

2. A method according to Claim 1, **characterized in that** a conductor model is formed of the brain (2), which is used to calculate the induced electrical field created in the brain (2).

3. A method according to Claim 1 or 2, **characterized in that** the magnitude or quality of the physiological or biological response is displayed in relation to the location of the maximum of the electromagnetic fields.

4. A method according to any of the above Claims, **characterized in that** the positions of both the coil (4) creating the electromagnetic field and of the head (3) are measured wirelessly, for example, using infrared technology (7) and a three-dimensional model of the position in the brain (2) of the electromagnetic field in at least more or less real time with the aid of the position data.

5. A method for targeting stimulation at a point in the brain defined by the operator
**characterized in that**

    - the desired point in the brain (2) is indicated from a magnetic image,

    - the optimal location and angles of tilt, relative to the head (3), of the coil (4) that acts as the excitation component are calculated in order to create the maximum stimulation in the target point inside the head,

    - an interactive computer program guides the operator to bring the coil (4) to the optimal location and attitude.

6. A method for the precise charting of responses to magnetic stimulation, such as, for example, a muscular response measured using electromyography, **characterized in that**

    - the coil (4) is located relative to the head (3) by using positioning devices (7, 8) immediately after each stimulation,

    - the electrical field created in the head (3) and the area of the strongest effect of the field are calculated,

    - some physiological or biological response is measured or detected,

    - the magnitude or quality of the response is depicted as a chart in the point of the strongest effect of the stimulation field created in the brain by the coil (4).

7. A method according to any of the above Claims, **characterized in that** the operator is guided in such a way that the same, or nearly the same distribution of the electrical field is created in the brain (2) on different stimulation occasions, and that point of the strongest effect of the stimulation is at the same location.

8. A method according to any of the above Claims, **characterized in that** the method is used for charting/mapping, in which the position of a specific function or structure in the brain is determined.

9. A method according to any of the above Claims, **characterized in that** a point, which is on the scalp of the person and which is closest to the target point in the brain cortex defined by the operator from an MR image, is defined as the optimal position of a symmetrical figure-of-eight coil.

10. A method according to any of the above Claims, **characterized in that** the optimal location and attitude of the coil (4) are calculated in such a way that the point, at which when the coil (4) is placed it creates the largest possible electrical field when the characteristics of the magnetic field induced by the coil are kept constant, is selected.

11. A method according to any of the above Claims, **characterized in that** the magnitude or quality of a measured physiological response are used as feedback, when calculating the optimal attitude, location, and/or stimulation strength of the stimulation coil.

12. A user interface in connection with electromagnetic stimulation, **characterized in that** at least a more or less real-time three-dimensional model of the brain (2) and of the electromagnetic radiation directed to the brain is depicted in the interface with the aid of a two-dimensional display (9).

13. An apparatus for targeting electromagnetic stimulation onto the human brain, which apparatus includes

- means (4) for creating such pulse-like electromagnetic fields as have a powerful local electromagnetic maximum,
- means for targeting the electromagnetic maximum of the field on the brain,
- means for determining the distribution, direction, and strength of the electromagnetic field created in the brain, and
- means for measuring a physiological or biological response caused by the stimulation,

**characterized in that** the apparatus includes

- means (5, 6, 7) for defining the position of the brain in three-dimensional space and for receiving the position data in the data system,
- means (7) for receiving the position data of the electromagnetic fields,
- means (8) for modelling the position of the electromagnetic fields together with the position of the brain (2) in the same three-dimensional set of co-ordinates, in at least more or less real time, and
- means (9) for forming a graphical presentation, of the position data of the electromagnetic fields and of the position data of the brain, with the aid of the data system.

14. An apparatus according to Claim 13, **characterized in that** it includes means (8) for forming a conductor model of the brain, which model can be used to calculate the induced electrical field created in the brain.

15. An apparatus according to Claim 13 or 14, **characterized in that** it includes means for displaying the magnitude or quality of a physiological or biological response in relation to the location point of the maximum of the electromagnetic fields.

16. An apparatus according to any of the above Claims, **characterized in that** it includes a coil calibration element, with the aid of which, by using a positioning device, it is possible to determine the exact location and attitude, relative to the coil, of a positioning element attached to a known coil of any geometry.

17. An apparatus according to any of the above Claims, **characterized in that** the apparatus includes means for using the magnitude or quality of a measured physiological response as feedback, when calculating the optical location, attitude, and/or stimulation strength of the stimulation coil.

Fig. 2

Fig. 1

Fig. 3

Fig. 4

**European Patent Office**

## PARTIAL EUROPEAN SEARCH REPORT

which under Rule 45 of the European Patent Convention shall be considered, for the purposes of subsequent proceedings, as the European search report

**Application Number**

EP 03 39 6048

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| E | EP 1 273 320 A (BRAINLAB AG) 8 January 2003 (2003-01-08) * column 2, line 31 - column 3, line 45 * * column 4, line 11 - line 50 * * column 5, line 12 - line 31 * * column 5, line 43 - column 6, line 24 * * column 8, line 10 - column 9, line 43 * ----- | 12-17 | A61N2/02 A61N2/00 |

**TECHNICAL FIELDS SEARCHED (Int.Cl.7)**

A61N

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

12-17

Claims searched incompletely :

Claims not searched :

1-11

Reason for the limitation of the search:

Article 52 (4) EPC - Method for treatment of the human or animal body by therapy

| Place of search | Date of completion of the search | Examiner |
|---|---|---|
| MUNICH | 1 August 2003 | Beck, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EP 1 366 782 A1

**ANNEX TO THE EUROPEAN SEARCH REPORT
ON EUROPEAN PATENT APPLICATION NO.**

EP 03 39 6048

This annex lists the patent family members relating to the patent documents cited in the above-mentioned European search report.
The members are as contained in the European Patent Office EDP file on
The European Patent Office is in no way liable for these particulars which are merely given for the purpose of information.

01-08-2003

| Patent document cited in search report | Publication date | Patent family member(s) | Publication date |
|---|---|---|---|
| EP 1273320 A | 08-01-2003 | EP 1273320 A1 | 08-01-2003 |
| | | EP 1270043 A1 | 02-01-2003 |
| | | EP 1269913 A1 | 02-01-2003 |
| | | US 2003004392 A1 | 02-01-2003 |
| | | US 2003023159 A1 | 30-01-2003 |
| | | US 2003065243 A1 | 03-04-2003 |